# EUROPEAN PATENT SPECIFICATION

(11) **EP 1 672 037 B1**
(45) Date of publication and mention of the grant of the patent: **07.12.2016**
(21) Application number: 05027481.0
(22) Date of filing: 15.12.2005
(51) Int. Cl.: C09C 1/00, C09D 11/00, C09D 11/16, C09D 11/02, A61K 8/26, A61Q 1/02, A61K 8/02, A61K 8/19, C09D 5/36, C09D 11/037

(54) **PEARLESCENT PIGMENT**
PERLGLANZPIGMENT
PIGMENT NACRÉ

(30) Priority: 16.12.2004 JP 2004363964; 16.12.2004 JP 2004363965
(43) Date of publication of application: 21.06.2006
(73) Proprietor: KAO CORPORATION, Tokyo 103-8210 (JP); Nihon Koken Kogyo KK, Tachikawa-shi Tokyo 190-0033 (JP)
(72) Inventor: Misaki, Eiichiro, Tokyo 131-8501 (JP); Shiomi, Hiroyuki, Haga-gun Tochigi 321-3497 (JP); Adachi, Maoya c/o Nihon Koken Kogyo KK, Tachikawa-shi Tokyo 190-0033 (JP); Kaida, Takahiro c/o Nihon Koken Kogyo KK, Tachikawa-shi Tokyo 190-0033 (JP)
(74) Representative: Hoffmann Eitle

(56) References cited:
- EP-A- 0 589 681
- EP-A- 0 914 387
- DE-A1- 4 104 310
- JP-A- 8 259 840
- US-A- 5 972 098
- US-A- 6 001 373

## Description

### FIELD OF THE INVENTION

The present invention relates to a novel pearl pigment, a process for producing the same, and make-up cosmetics containing the same.

### BACKGROUND OF THE INVENTION

There have been used a variety of coloring pigments in areas such as coatings, plastics, printing inks and cosmetics. Examples of such coloring pigments include: inorganic pigments such as iron oxide pigments, Prussian blue, ultramarine and chromium oxide; and organic pigments such as Red 201, yellow 401, Blue 1 and Green 3. Generally, some of them are used in combination. However, such coloring pigments have the disadvantage that when toning using a plurality of the coloring pigments, a darker color is produced due to their light absorption.

Thus, there have been used light-interference pearl pigments which are obtained by coating the surface of flaky powder with titanium oxide, iron oxide or the like to materialize bright color. Of these light interference pearl pigments, light absorption does not occur in pearl pigments obtained by coating a flakypowderwith a colorless metal oxide, such as titanium oxide, whereas in pearl pigments obtained by coating a flaky powder with a colored metal oxide, such as iron oxide, light absorption occurs and they have a characteristic of producing a darker color due to their light absorption, just like commonly used coloring pigments such as iron oxide pigments or organic pigments. There have been proposed a variety of processes for coating a flaky powder with a metal oxide, including iron oxide and there are a variety of powders on the market; however, those existing pearl pigments are all such that they each produce a color of a similar shade to that of the color compound used, for example, pearl pigments obtainedusing iron oxide eachhave a color of a reddish shade, because of light scattering caused by the fine irregularities of the pigment surface.

As described in, for example, JP-A-49-128027, pigments obtained by coating the surface of mica with an iron oxide layer have long been examined. And processes, such as the homogenous precipitation method or neutralization titration method in which an iron salt aqueous solution and an alkaline aqueous solution are introduced simultaneously into a dispersion of a flaky powder, have been used. However, in any pigments obtained by using iron oxide as a coating layer, their colors have been of a reddish shade inherent to iron. In JP-A-1-60511, there is described a pearl pigment to which an alkaline earth metal has been added so as to improve the clarity of the color produced. However, its coating is an acicular crystal, and moreover, examples of the specification state that the color of the pigment obtained is of a reddish shade. This indicates that light absorption due to light scattering has not been sufficiently retarded.

JP-A-10-259318 describes an orange pearl pigment having a coating layer of a metal oxide to which an aluminum oxide has been added. However, the color of the pigment is orange of a reddish shade, which indicates that light absorption due to light scattering is not sufficiently retarded. Further, there are pearl pigments having a coating layer to which an aluminum oxide has been added, and for example, Colorona Passion Orange by Merck, are on the market. In these pigments, too, light absorption due to light scattering is not sufficiently retarded.

Meanwhile, of cosmetics using coloring pigments, make-up cosmetics are required to change users' skin texture when they apply them to their skin, and powders that give rise to strong diffuse reflection or specular reflection are used to control users' skin tone and texture. For example, titanium oxide as a powder that gives rise to strong diffuse reflection or a spherical powder is blended with make-up cosmetics to create a matte finish, or a platelet-shaped powder, such as mica, sericite or talc, which gives rise to strong specular reflection is blended with make-up cosmetics to impart a lustrous finish. To cover color non-uniformity such as liver spots or freckles, pigments having good hiding power such as titanium dioxide or iron oxide are also blended with make-up cosmetics.

However, the use of pigments with good hiding power causes a problem of producing an unnatural look, whereas the use of powders with poor hiding power to adjust the skin tone and texture causes a problem of decreasing covering effect on the color non-uniformity, etc. of the skin.

To overcome the above described problems, titanium oxide-coated pearl pigments that have good hiding power and are capable of controlling skin luster have been used for changing the hue of the skin, in recent years. For example, when a titanium-oxide coatedpearl pigment is used in cosmetics for imparting a lustrous finish to the skin, skin luster can be produced in such a direction that regular reflection of light occurs, due to the specular reflection by the pigment, but not through an angle other than that of specular reflection, and at such an angle the cosmetics impart a whitish look of the powder itself. Accordingly, when a user applies the cosmetics over a wide range, for example, to the entire face, the portion directly facing its observer provides a lustrous look, but the portion not directly facing its observer provides a whitish and powdery look, resulting in a tendency toward an unnatural finish. Further, Japanese Patent Publication JP-A-2003-212722 describes a powder for producing natural skin luster which is obtained by coating the surface of titanium mica with alumina and silica in this order.

On the other hand, there are pearl pigments in which a coating is applied onto the colored metal oxide layer, such as iron oxide, on the market; however, since they are pigments each having a color of a reddish shade, they can impart luster of a reddish shade to the skin, but on the other hand, such skin gives an unnatural impression on those who see it.

JP 08-259840 discloses a red-based pigment comprising a platelet-shaped particle that is covered with an iron oxide layer, wherein the surface of the iron oxide layer is covered with a titanium compound layer containing a titanium dioxide, the iron oxide layer has an optical thickness of 180 to 320 nm. The pigment is produced by coating the platelet-shaped particle with the iron oxide so that the amount of the iron oxide, is 80 to 140 wt% in relative to the amount of the platelet-shaped particle using a precipitation method from a solution of an iron oxide precursor, which is subsequently coated with the titanium compound layer and baked at 300 to 900°C. The pigment thus produced is useful for cosmetics application.

DE 4104310 discloses a platelet-shaped pigment comprising a platelet-shaped metal substrate that is covered with one or more metal oxide, wherein the metal oxide is iron oxide. The pigment is produced by treating an aqueous suspension of the platelet-shaped metal substrate with an iron salt solution at constant pH, preferably 4 to 10, separating the thus-coated metal substrates and then optionally washing, drying .and/or annealing. The pigment is useful for cosmetics application.

US 5,972,098 discloses titanate-containing pearlescent comprising iron titanate and optionally titanium oxide and/or iron oxide, which is produced by obtaining titanium dioxide platelets, and coating the thus obtained platelets with iron oxide in a wet process, then drying and calcing.

### SUMMARY OF THE INVENTION

The present invention provides a pearl pigment having a flaky powder coated on the surface with a colored metal or a coloredmetal oxide, wherein when the pearl pigment is applied onto the surface of black artificial leather in an average amount of 0. 05 mg/cm² and the intensity of light reflected from the pearl pigment is measured using illuminant C at a 2° viewing angle with a spectrophotometric colorimeter which is provided with a sheet polarizer S on the light incident side and a sheet polarizer P on the light receiving side, the reflection light (scattered light) of the pigment, which is incident to the pearl pigment as a measurement sample at an angle of 45° to the direction normal to the surface of the sample and is received in the direction normal to the surface of the sample has absolute values a* and b* of 5 or less.

According to the present invention, the average surface roughness of the coating is 10 nm or less. The flaky powder and the colored metal (oxide) are defined in claim 1.

Further, the present invention provides a process for producing a pearl pigment which includes: adding an aqueous solution of a colored metal oxide precursor to an aqueous dispersion of a flaky powder so that the amount of metal ion is 5 × 10⁻⁴ to 12 × 10⁻⁴ mol/min per 100 g of the flaky powder; adding an alkaline solution to the mixed solution so that the pH of the mixed solution is adjusted to 5 to 8; separating the solid from the mixed solution and baking the separated solid at 500 to 1000°C.

Still further, the present invention provides make-up cosmetics that contain any one of the above described pearl pigments.

### BRIEF DESCRIPTION OF THE DRAWINGS

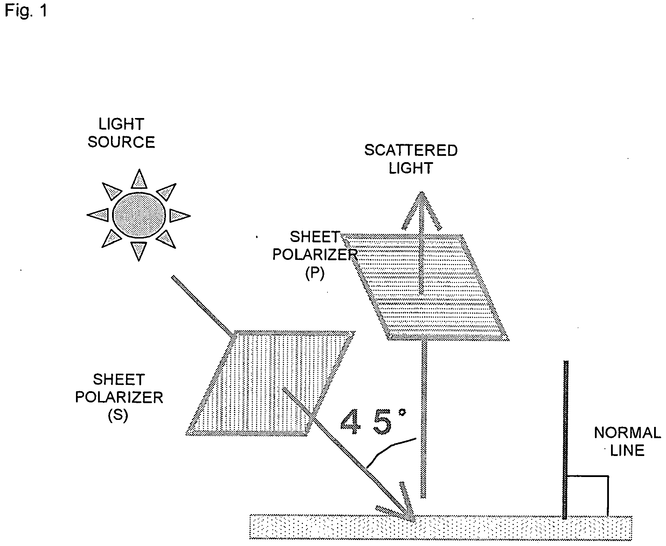

FIG. 1 illustrates "the method for measuring the value a* and value b* of the present invention".

### DETAILED DESCRIPTION OF THE INVENTION

The present invention relates to: a novel pearl pigment which retards the production of scatter colors; a process for producing such a pearl pigment; and make-up cosmetics which contain the pearl pigment.

In the present invention, value a* and value b* each indicate chromaticity standardized by Commission International de L'Eclairage (CIE) (1976). The method for measuring the values a* and b* of each pearl pigment herein shown (see FIG. 1) is referred to as "the method for measuring the values a* and b* of the present invention".

The present inventors have found that a pearl pigment obtained by coating the surface of a flaky powder with a colored metal or a colored metal oxide, wherein the absolute values of the values a* and b* of the scattered light measured under specified conditions are 5 or less, produces brilliant colors and that the use of such a pearl pigment makes it possible to obtain make-up cosmetics capable of changing the hue of the skin while imparting a sheer dewy finish to the skin, creating a light smooth finish and producing a natural luster on the skin; thus, they have finally accomplished the present invention.

The pearl pigment of the present invention produces brilliant colors while retarding the production of scatter colors and producing only a small quantity of scattered light. The make-up cosmetics of the present invention that contain the above described pearl pigment are capable of changing the hue of the skin while imparting a sheer dewy finish to the skin, creating a light smooth finish and producing a natural lusteron the skin. Their feeling in use, such as spreadability when applied to the skin, is also good.

The flaky powder used in the pearl pigment of the present invention is preferably 2 to 200 µm in average particle size and 0.01 to 5 µm in average thickness and, from the viewpoint of suitability for blending, more preferably 2 to 20 µm in average particle size and 0.05 to 1 µm in average thickness. The term "average particle size" herein used means volume average particle size (D4) (average particle size calculated based on volume fraction). The measurement can be made with ease and good reproducibility using a laser diffraction particle size distribution analyzer. For the thickness of the flaky particle, the difference from the reference surface is measured with an atomic force microscope and the arithmetic mean of the measurements is used as an average thickness.

The flaky powder is mica, sericite, talc, kaolin, smectite clay mineral, synthetic mica, synthetic sericite, platelet-shaped titanium dioxide, platelet-shaped silica, platelet-shaped aluminum oxide, boron nitride, barium sulfate, platelet-shaped titania-silica composite oxide. As the flaky powder, mica is preferable in terms of surface smoothness.

The colored metal is gold or copper. As the colored metal, gold is preferable. The colored metal oxide is iron oxide, lower titanium oxide, copper oxide, cobalt oxide, chromium oxide or nickel oxide. As the colored metal oxide, iron oxide is preferable.

The pearl pigment of the present invention is such that the values a* and b* of the light reflected from the pigment powder, which are measured in accordance with "the method for measuring the values a* and b* of the present invention", are 5 or less and preferably 3 or less.

As a measurement instrument applicable to such colorimetry, color measurement system GCMS series manufactured by Murakami Color Research Laboratory Co., Ltd. can be used.

The pearl pigment of the present invention is obtained by coating the surface of a flaky powder with a colored metal or colored metal oxide, as described above, and the average surface roughness of the colored metal or colored metal oxide is 10 nm or less and preferably 5 nm or less. Since colored metal oxides have properties of absorbing light having particular wavelengths, the larger the roughness of the coating surface becomes, the more intense the scatter color becomes, resulting in the production of color of a low pearly luster. Decreasing the roughness of the coating surface retards the scattering of light, which makes it possible to obtain a pearl pigment producing less scattered light and hence more brilliant colors.

In the present invention, the term "average surface roughness (Ra)" indicates arithmetic average roughness, which is an average value of the measurements obtained by measuring the range of 2 µm × 2 µm using an atomic force microscope (Nanoscope III by Digital Instrument) with scan rate set to 1.0 Hz. When the measurement is made, the dispersion of the pearl pigment in a solvent such as ethanol is deposited on a flat base and then the solvent is removed to allow the pigment to closely attach to the base. The measurement is then made with an atomic force microscope.

In the pearl pigment of the present invention, the optical thickness of the coating layer is preferably 250 nm or less, and more preferably 210 nm or less. If the optical thickness is larger than 250 nm, the absolute thickness of the absorption layer is increased, the effect of light absorption is also increased, and the effect of sufficiently suppressing the scattered light may be reduced.

The optical thickness of the colored metal or colored metal oxide coating layer herein used means the thickness obtained by multiplying the geometric thickness of the coating layer by the refractive index of the same. In case of iron oxide, the geometric thickness of the coating layer is preferably 80 nm or less and more preferably 50 nm or less. The geometric thickness of the coating layer is determined with a SEM.

When the optical thickness of the coating layer is in the range of 120 to 210 nm, the pigment produces a gold interference light, whereas when the optical thickness of the coating layer is in the range of 15 to less than 120 nm and preferably 60 to less than 120 nm, the pigment produces a silver interference light. Thus the colors produced are aesthetic and preferable. If the scatter color is intense as in the case of conventional pearl pigments, the interference light is killed by the scatter color and is invisibly to the naked eye. In the pearl pigment of the present invention, since the scatter color is retarded, interference light having colors other than those characteristic of the coated colored metal, such as gold or silver, can also be produced.

When such a pearl pigment is used in make-up cosmetics, the portion directly facing observers produces a lustrous look, whereas the portion not directly facing observers does not produce a whitish and powdery look, unlike titanium oxide-coated mica, and naturally produces a dark red shade, but imparts a more natural skin like finish.

The pearl pigment of the present invention can be produced by selecting the rate of adding an aqueous solution of a colored metal oxide precursor, for example, using the neutralization titration method. The neutralization titration method is preferably used for decreasing the surface roughness of the colored metal or colored metal oxide.

Specifically, the pearl pigment of the present invention can be produced by: adding an aqueous solution of a precursor of a colored metal oxide to an aqueous dispersion of a flaky powder so that the amount of metal ion is 5 × 10⁻⁴ to 12 × 10⁻⁴ mol/min per 100 g of the flaky powder; adding an alkaline solution to the mixed solution so that the pH of the mixed solution is 5 to 8; separating solid from the mixed solution; baking the solid at 500 to 1000°C.

First, a flaky powder is dispersed in water and fully stirred to prepare an aqueous dispersion of the flaky powder. The slurry concentration of the dispersion is preferably 1 to 50% by weight for coating the surface of the flaky powder uniformly with a metallic compound.

Examples of precursors of a colored metal oxide include ferric nitrate, ferric chloride and ferric sulfate. The concentration of the aqueous solution of such a precursor is preferably 20 to 70% by weight.

The dispersion of the flaky powder is heated to 50 to 100°C and preferably to 70 to 80°C, an acid is added to the dispersion to give an acidic dispersion, and an aqueous solution of a precursor of a colored metal oxide is added to the reactionmixed solution while keeping the pH of the reaction solution at 2 to 4 and preferably 2.5 to 3.5 by adding an alkaline aqueous solution. To realize a smooth coated state in which surface roughness is minimal, the aqueous solution of a precursor of a colored metal oxide is added at a rate that allows the amount of metal ion to be 5 × 10⁻⁴ to 12 × 10⁻⁴ mol/min per 100 g of the flaky powder and preferably 8 × 10⁻⁴ to 11 × 10⁻⁴ mol/min per 100 g of the flaky powder. If the rate of adding the aqueous solution of a precursor of a colored metal oxide is in such a range, the scattered light is retarded, and the resultant pigment can be a preferable one.

Examples of alkaline aqueous solutions used for pH adjustment include: aqueous solutions of sodium hydroxide, potassium hydroxide, sodium carbonate, potassium carbonate, sodium hydrocarbonate and potassium hydrocarbonate.

After completing the addition, the mixed solutionis aged. Then, an alkaline aqueous solution is added to adjust the pH of the mixed solution to 5 to 8, and the solution is further aged. After separating the solid, the solid is rinsed to remove the salt and dried. The dried solid is then baked at 500 to 1000°C and preferably at 700 to 800°C for 30 to 180 minutes, so that the pearl pigment of the present invention can be obtained.

The pearl pigment of the present invention can be used as a coloring material for, for example, cosmetics, coatings, printing inks, plastics, ceramics, or glaze for glasses.

Themake-up cosmetics of the present inventionmay contain the pigment described above. The surface of the pearl pigment can undergo hydrophobic treatment before use, from the viewpoint of improving the tenacity of the cosmetics. Hydrophobic treatment can be carried out by a conventional process using a hydrophobic treatment agent.

Examples of hydrophobic treatment agents which may be used include: silicone oil, fatty acid metal salt, alkyl phosphoric acid, alkali metal salts or amine salts of alkyl phosphoric acid, N-mono long chain (with 8 to 22 carbon atoms) aliphatic acyl-basic amino acid, and fluorine compound having a perfluoroalkyl group.

Examples of silicone oils which may be used include: various types of chain silicone, cyclic silicone and modified silicone. Examples of metal salts of fatty acids used include: salts of fatty acids having 12 to 18 carbon atoms and calcium, magnesium, zinc, aluminum, or the like. Examples of alkyl phosphoric acids and the salts thereof used include: mono-or di-esters containing alkyl or alkenyl group having 8 to 45 carbon atoms in total, or alkali metal salts or amine salts thereof. Examples of N- mono long chain aliphatic acyl-basic amino acids include: basic amino acids whose amino group at α- or ω-position is bound with acyl group having 8 to 22 carbon atoms, such as 2-ethyl hexanoyl, capryloyl, lauroyl, myristoyl, palmitoyl, stearoyl, isostearoyl, oleoyl, behenoyl, cocoyl, beef tallow fatty acid acyl or hydrogenated beef tallow fatty acid acyl. Examples of fluorine compounds containing a perfluoroalkyl group include: those described in U.S Patent No. 3632744, JP-A-62-250074, JP-A-55-167209 and JP-A-2-218603.

In the hydrophobic treatment, the amount of hydrophobic treatment agent used is preferably 0.0005 to 0.2 parts by weight and more preferably 0.02 to 0.1 parts by weight based on 1 part of pearl pigment, from the viewpoint of imparting sufficient hydrophobicity and a good feel to make-up cosmetics.

For the pearl pigments described above, more than one type can be used in combination. The amount of the pearl pigments contained in make-up cosmetics is preferably 0.1 to 30% by weight and more preferably 0.5 to 30% by weight, from the viewpoint of allowing the make-up cosmetics to fully produce the blend effect and of avoiding an unnatural look due to too much luster. The pearl pigment of the present invention can also be used in combination with conventionally used pearl pigments.

The make-up cosmetics of the present invention can be produced by conventional processes. They can be, for example, a powdery face powder, cake-type face powder, face powder, powdery foundation, oil-based foundation, cream foundation, liquid foundation, concealer, lip rouge, lip cream, cheekrouge, eye shadow, eye liner or eyebrow pencil.

Preferably, the amount of the pearl pigment of the present invention contained in the make-up cosmetics of the present invention is: 0.1 to 30% by weight and more preferably 0.5 to 30% by weight, when the cosmetics are powdery face powder, cake-type face powder and face powder; 0.1 to 30% by weight and more preferably 0.5 to 30% by weight, when the cosmetics are powdery foundation and oil-based foundation; 0.1 to 30% by weight and more preferably 0.5 to 25% by weight, when the cosmetics are cream foundation, liquid foundation and concealer; 0.1 to 20% by weight and more preferably 0.5 to 15% by weight, when the cosmetics are lip rouge and lip cream; 0.1 to 30% by weight and more preferably 0.5 to 25% by weight, when the cosmetics are cheek rouge and eye shadow; and 0.1 to 30% by weight and more preferably 0.5 to 20% by weight, when the cosmetics are eye liner and eyebrow pencil.

The make-up cosmetics of the present inventionmay further include a lubricant whose viscosity is 1000 mPa·s or less at 25°C. From the viewpoint of feeling in use, the viscosity of the lubricant used is preferably in the range of 0.1 to 1000 mPa·s and more preferably in the range of 2 to 500 mPa·s.

Any lubricant can be used as long as its viscosity is 1000 mPa·s or less. Examples of such lubricants include: liquid paraffin, squarane, olive oil, ester oil, diglyceride, triglyceride, silicone oil and fluorine lubricant having a perfluoroalkyl group. Of these lubricants, liquid paraffin, squarane, esteroil, silicone oil and fluorine lubricant having a perfluoroalkyl group are preferably used.

More than one type of lubricant can be used in combination. The amount of the lubricants used is preferably 0.5 to 60% by weight and more preferably 2 to 50% by weight per 100% of make-up cosmetics, from the viewpoint of being free.from a sticky feeling when used.

To allow make-up cosmetics to have preferable spreadability and show expected optical characteristics when they are applied to the skin, the blending ration of the pearl pigment to lubricants is preferably 1/10 to 100/1.

The make-up cosmetics of the present invention may include, if necessary, ingredients blended in commonly used cosmetics, such as surfactant, water-soluble polymer, other powders, moisturizer, preservative, chemical, UV absorber, pigment, inorganic or organic salt, perfume, chelating agent, pH adjuster or water, besides the above described ingredients.

### [Examples]

The following examples further describe and demonstrate embodiments of the present invention. The examples are given only solely for the purpose of illustration and are not to be construed as limitations of the present invention.

### Example 1

100 g of flaky mica 5 to 60 µm in particle size were added to and fully dispersed in 1.5 L of water, the mica dispersion was heated to 80°C, and hydrochloric acid was added so that the pH of the dispersion was adjusted to 3. Then, 421 g of previously prepared ferric nitrate aqueous solution (17 parts by weight of ferric nitrate : 26 parts by weight of water) were added slowly, at a rate of 8.6 × 10⁻⁴ mol iron ion concentration/min, to the mica dispersion while keeping the pH of the dispersion at 3 using a sodium hydroxide aqueous solution. After completing the addition, the pH of the mixed solution was adjusted to 5 by adding a sodium hydroxide aqueous solution to the mixed solution. The pH-adjusted solution was filtered to obtain a solid, and the obtained solid was rinsed to remove the salt, suction filtered, dried, and baked at 700°C for 1 hour.

The resultant pearl pigment was such that a coating layer of very fine iron oxide particles 20 nm in geometric thickness was formed uniformly on the surface of the flaky mica. The surface roughness of the coating layer measured 3.44 nm.

### Example 2

A pearl pigment was produced in the same manner as Example 1, provided that the amount of the ferric nitrate aqueous solution (17 parts by weight of ferric nitrate : 26 parts by weight of water) added was changed from 421 g to 538 g.

The obtained pearl pigment was such that a coating layer of very fine iron oxide particles 30 nm in geometric thickness was formed uniformly on the surface of the flaky mica. The surface roughness of the coating layer measured 4.30 nm.

### Example 3

A pearl pigment was produced in the same manner as Example 1, provided that the amount of ferric nitrate aqueous solution (17 parts by weight of ferric nitrate : 26 parts by weight of water) added was changed from 421 g to 680 g.

The obtained pearl pigment was such that a coating layer of very fine iron oxide particles 50 nm in geometric thickness was formed uniformly on the surface of the flaky mica. The surface roughness of the coating layer measured 3.03 nm.

### Example 4

A pearl pigment was produced in the same manner as Example 1, provided that the amount of ferric nitrate aqueous solution (17 parts by weight of ferric nitrate : 26 parts by weight of water) added was changed from 421 g to 914 g.

The obtained pearl pigment was such that a coating layer of very fine iron oxide particles 80 nm in geometric thickness was formed uniformly on the surface of the flaky mica. The surface roughness of the coating layer measured 3.92 nm.

### Example 5

A pearl pigment was produced in the same manner as Example 1, provided that the iron ion concentration of the ferric nitrate aqueous solution (17 parts by weight of ferric nitrate: 26 parts by weight of water) was changed to that of the ferric nitrate aqueous solution, (17 parts by weight of ferric nitrate: 7 parts by weight of water) and the amount of the ferric nitrate aqueous solution added was changed to 241 g. The rate of adding the ferric nitrate aqueous solution was 8.6 × 10⁻⁴ mol iron ion concentration/min.

The obtained pearl pigment was such that a coating layer of very fine iron oxide particles 20 nm in geometric thickness was formed uniformly on the surface of the flaky mica. The surface roughness of the coating layer measured 4.48 nm.

### Example 6

A pearl pigment was produced in the same manner as Example 1, provided that the iron ion concentration of the ferric nitrate aqueous solution (17 parts by weight of ferric nitrate: 26 parts by weight of water) was changed to that of the ferric nitrate aqueous solution (17 parts by weight of ferric nitrate: 68 parts by weight of water) and the amount of the ferric nitrate aqueous solution addedwas changed to 842 g. The rate of adding the ferric nitrate aqueous solution was 8.6 × 10⁻⁴ mol iron ion concentration/min.

The obtained pearl pigment was such that a coating layer of very fine iron oxide particles 20 nm in geometric thickness was formed uniformly on the surface of the flaky mica. The surface roughness of the coating layer measured 3.69 nm.

### Example 7

1.3 kg of flaky mica 5 to 60 µm in particle size were added to and fully dispersed in 20 L of water, the mica dispersion was heated to 80°C, and hydrochloric acid was added so that the pH of the dispersion was adjusted to 3. Then, 5.6 kg of previously prepared ferric nitrate aqueous solution (17 parts by weight of ferric nitrate : 26 parts by weight of water) were added slowly, at a rate of 1.4 × 10⁻² mol iron ion concentration/min (10.7 × 10⁻⁴ mol/min for 100 g of mica), to the mica dispersion while keeping the pH of the dispersion at 3 using sodium hydroxide aqueous solution. After completing the addition, the pH of the mixed solution was adjusted to 5 by adding a sodium hydroxide aqueous solution to the mixed solution. The product was filtered, rinsed to remove the salt, filtered by suction, dried, and then baked at 700°C for 1 hour.

The obtained pearl pigment was such that a coating layer of very fine iron oxide particles 20 nm in geometric thickness was formed uniformly on the surface of the flaky mica. The surface roughness of the coating layer measured 6.96 nm.

### Example 8

A pearl pigment was produced in the same manner as Example 1, provided that the amount of ferric nitrate aqueous solution (17 parts by weight of ferric nitrate : 26 parts by weight of water) added was changed from 421 g to 180 g.

The obtained pearl pigment was such that a coating layer of very fine iron oxide particles 10 nm in geometric thickness (30 nm in optical thickness) was formeduniformlyonthe surface of the flaky mica. The surface roughness of the coating layer measured 8.7 nm.

### Example 9

A pearl pigment was produced in the same manner as Example 1, provided that the amount of ferric nitrate aqueous solution 420 g (17 parts by weight of ferric nitrate : 26 parts by weight of water) added was changed from 421 g to 84 g.

The obtained pearl pigment was such that a coating layer of very fine iron oxide particles 5 nm in geometric thickness (15 nm in optical thickness) was formed uniformly on the surface of the flaky mica. The surface roughness of the coating layer measured 3.8 nm.

### Comparative Example 1

120 g of flaky mica 5 to 60 µm in particle size were added to 820 g of water and heated to 95°C. And a solution of 350 g of urea, 170 g of ferrous sulfate, 10 g of ferric nitrate and 7 g of 30% nitric acid aqueous solution in 308 g of water was added dropwise to the above mica dispersion at a rate of about 25 g/min (19 x 10⁻³ mol/min) over 35 minutes. Then the mixed solution was stirred for 2 hours, and 205 g of 30% potassium carbonate aqueous solution were added dropwise to the mixed solution over about 40 minutes. Then, the solution was rinsed to remove the salt, suction filtered, dried, and baked at 700°C for 1 hour.

The obtained pigment was such that a coating layer of about 0.1 µm iron oxide particles 30 nm in geometric thickness was formed a little roughly on the surface of the flaky mica. The surface roughness of the coating layer measured 13.41 nm.

### Comparative Example 2

A pearl pigment was obtained in the same manner as Comparative Example 1, provided that the amount of the urea in the iron salt solution added was changed from 350 g to 370 g, the amount of the ferrous sulfate in the same from 170 g to 200 g, the amount of the ferric nitrate in the same from 10 g to 12 g and the rate of adding the iron salt solution was changed to 21 × 10⁻³ mol/min.

The obtained pigment was such that a coating layer of very fine iron oxide particles 50 nm in geometric thickness was formed a little roughly on the surface of the flaky mica. The surface roughness of the coating layer measured 13.52 nm.

### Comparative Example 3

A pearl pigment was obtained in the same manner as Comparative Example 1, provided that the amount of the urea in the iron salt solution added was changed from 350 g to 390 g, the amount of the ferrous sulfate in the same from 170 g to 300 g, the amount of the ferric nitrate in the same from 10 g to 16 g, the amount of water from 308 g to 320 g and the rate of adding the iron salt solution was changed to 27 × 10⁻³ mol/min.

The obtained pigment was such that a coating layer of very fine iron oxide particles 80 nm in geometric thickness was formed a little roughly on the surface of the flaky mica. The surface roughness of the coating layer measured 10.24 nm.

### Comparative Example 4

A pearl pigment was produced in the same manner as Example 2, provided that the rate of adding the previously prepared ferric nitrate aqueous solution was increased from 8.6 × 10⁻⁴ mol/min to 14 × 10⁻⁴ mol/min.

The obtained pigment was such that a coating layer of very fine iron oxide particles 30 nm in geometric thickness was formed a little roughly on the surface of the flaky mica. The surface roughness of the coating layer measured 14.05 nm.

### Comparative Example 5

A pearl pigment was produced in the same manner as Example 2, provided that the rate of adding the previously prepared ferric nitrate aqueous solution was decreased from 8. 6 × 10⁻⁴ mol/min to 3.75 × 10⁻⁴ mol/min.

The obtained pigment was such that a coating layer of very fine iron oxide particles 30 nm in geometric thickness was formed a little roughly on the surface of the flaky mica. The surface roughness of the coating layer measured 10.68 nm.

### Test examples

The values a* and b* of powder reflection light (scattered light) were determined for the pearl pigments obtained in Examples 1 to 9 and Comparative Examples 1 to 5 and commercially available iron oxide-coated mica (IRIODIN 504 Red (Merck): Commercially available product 1; Colorona Passion Orange (Merck) : Commercially available product 2) in accordance with "the method for measuring the values a* and b* of the present invention". As a spectrophotometric colorimeter, GCMS-4, manufactured by Murakami Color Research Laboratory Co., Ltd., provided with sheet polarizer (Polaroid, Type: HN32). As a light source, a halogen lamp (HLWS7) by NARVA Speziallampen GmbH was used. Measurement samples were prepared by using pieces of black artificial leather (Okamoto, Type: OK-7) and coating the area 5 cm × 10 cm of each piece with each pigment powder using sponge so that the amount of the coating applied was on average 0.05 mg/cm². The geometric thickness of each coating layer was measured with SEM. The geometric thickness was obtained by first measuring the thickness of the flaky powder before coating and then measuring the thickness of the pearl pigment after coating. The results are shown in Table 1.

**[Table 1]**

| | Value a* | Value b* | Outside Color of Coated Sample | Geometric Thickness (nm) | Optical Thickness (nm) |
|---|---|---|---|---|---|
| Example 1 | 1.96 | 0.55 | silver | 20 | 60 |
| Example 2 | 3.04 | 3.90 | somewhere between silver and gold | 30 | 90 |
| Example 3 | 3.72 | 3.04 | somewhere between gold and red | 50 | 150 |
| Example 4 | 3.04 | 0.98 | red | 80 | 240 |
| Example 5 | 3.28 | 4.13 | silver | 20 | 60 |
| Example 6 | 1.62 | 3.83 | silver | 20 | 60 |
| Example 7 | 0.41 | 2.72 | silver | 20 | 60 |
| Example 8 | 0.55 | 2.55 | Silver | 10 | 30 |
| Example 9 | 1.12 | 2.03 | silver | 5 | 15 |
| Comparative Example 1 | 8.56 | 9.01 | red | 30 | 90 |
| Comparative Example 2 | 12.99 | 9.90 | red | 50 | 150 |
| Comparative Example 3 | 11.39 | 8.24 | red | 80 | 240 |
| Comparative Example 4 | 2.05 | 5.33 | red | 30 | 90 |
| Comparative Example 5 | 6.06 | 3.60 | red | 30 | 90 |
| Commercially available pearl pigment 1 | 8.72 | 6.62 | red | - | - |
| Commercially available pearl pigment 2 | 8.88 | 7.57 | orange | - | - |

### Applications

A few examples of applications of the pearl pigments obtained in Examples, such as coatings, inks and plastics, willbeshownbelow. In each application, since a pearl pigment is mixed into a resin by kneading as generally known, each product has a hue different from that shown when the pigment powder by itself is coated on a product. This is why the color of the coated product in each application is shown in terms of its outside color.

### Application 1 (coating for automobiles)

A steel sheet with an intermediate coating applied to an electrodeposition coating was prepared. A color base coating was applied onto the surface of the intermediate coating, and the resultant steel sheet was baked at 140°C for 30 minutes to be dried. Then, a base coating was prepared which was a resin fluid-made up of acrylic resin and melamine resin and containing 5% by weight of pearl pigment obtained in Example 2 and spray coated on the surface of the above color base coating so that the coating thickness was 15 µm. Subsequently, an acryl-melamine clear coating was applied to the surface of the base coating by wet-on-wet spray coating so that the coating thickness was 40 µm, and the resultant steel sheet was baked at 140°C for 30 minutes so that the base coating and the clear coating was integrally cured. The coated steel sheet thus obtained had a brilliant bronze appearance.

### Application 2 (printing ink composition)

The ingredients shown in Table 2 were mixed, and the mixture was kneaded with a sand mill to obtain a printing ink composition.

Printing was performed on black paper using the obtained printing ink composition so that the thickness of the coating (after drying) was 50 µm. The coating had a brilliant orange appearance.

**[Table 2]**

| Ingredients | Content (parts by weight) |
|---|---|
| Pearl pigment of Example 3 | 12.5 |
| Acrylic resin | 25.0 |
| Naphtha | 30.0 |
| Butylcellosolve | 32.5 |

### Application 3 (plastic-coloring composition)

The ingredients shown in Table 3 were mixed with a Henschel mixer, and the obtained mixture was formed into colored pellets with an extruding machine. Then the pellets were formed into platelet-shaped pieces 90 × 50 × 2 mm in size in an extruder. The formed pieces had a brilliant red appearance.

**[Table 3]**

| Ingredients | Content (parts by weight) |
|---|---|
| Pearl pigment of Example 4 | 2.0 |
| Polyethylene resin | 97.5 |
| Dibutylhydroxytoluene (antioxidant) | 0.5 |

### Examples 10 to 12, Comparative Examples 6 to 7

Cake-type foundations each having the compositions shown in Table 4 were produced. The produced cake-type foundations were evaluated in terms of creating: a smooth finish, a light skin color, the smallest possible color difference between the forehead portion and the side cheek portion, and a natural luster. The evaluations are also shown in Table 4.

### (Production process)

The ingredients (1) to (12) were mixed while stirring, and then the ingredients (13) to (15) were added and mixed. Each mixture was pulverized with an atomizer and sieved, and the sieved powder was packed in a metal dish and compressed to obtain a cake-type foundation.

### (Evaluation method)

Twenty panels for cosmetics were allowed to use each of the produced cake-type foundations and scored points based on the criteria shown below. The quality of the foundations was judged using the average value of the points scored by the panels.
(1) creates a smooth finish, creates a light skin color, creates a natural luster;
   - 5 points:: very good
   - 4 points:: good
   - 3 points:: fair
   - 2 points:: a little poor
   - 1 point:: poor
(2) color difference between the forehead portion and the side cheek portion
   - 5 points:: almost no difference

   - 4 points:: small difference
   - 3 points:: somewhat difference
   - 2 points:: a little large difference
   - 1 point:: large difference

**[Table 4]**

| | Examples | | | Comparative Examples | |
|---|---|---|---|---|---|
| Ingredients (% by weight) | 10 | 11 | 12 | 6 | 7 |
| (1) Pearl pigment (Example 1) | 25 | | | | |
| (2) Pearl pigment (Example 2) | | 25 | | | |
| (3) Pearl pigment (Example 9) | | | 25 | | |
| (4) Talc | 20 | 20 | 20 | 20 | 20 |
| (5) Sericite | 16.5 | 16.5 | 16.5 | 41.5 | 16.5 |
| (6) Spherical nylon | 5 | 5 | 5 | 5 | 5 |
| (7) Zinc stearate | 3 | 3 | 3 | 3 | 3 |
| (8) Mica titanium* | | | | | 25 |
| (9) Titanium dioxide | 10 | 10 | 10 | 10 | 10 |
| (10) Yellow iron oxide | 7 | 7 | 7 | 7 | 7 |
| (11) Red iron oxide | 2 | 2 | 2 | 2 | 2 |
| (12) Black iron oxide | 1 | 1 | 1 | 1 | 1 |
| (13) Paraben | 0.5 | 0.5 | 0.5 | 0.5 | 0.5 |
| (14) Dimethyl polysiloxane (10 mPa·s) | 5 | 5 | 5 | 5 | 5 |
| (15) Liquid paraffin (10 mPa·s) | 5 | 5 | 5 | 5 | 5 |
| Creates a smooth finish | 4.7 | 4.7 | 4.6 | 4.5 | 1.3 |
| Creates a light skin color | 4.5 | 4.3 | 4.6 | 1.9 | 4.7 |
| Creates the smallest color difference between the forehead portion and the side cheek portion | 4.6 | 4.7 | 4.0 | 4.8 | 2.2 |
| Creates a natural luster | 4.7 | 4.7 | 4.7 | 3.0 | 2.2 |

| | | | | | |
|---|---|---|---|---|---|
| * TIMIRON MP-1005 (MERCK) | | | | | |

## Claims

1. A pearl pigment comprising a flaky powder coated on the surface with a colored metal or a colored metal oxide,
wherein when the pearl pigment is applied onto the surface of black artificial leather in an average amount of 0.05 mg/cm² and the intensity of light reflected from the pearl pigment is measured using illuminant C at a 2° viewing angle with a spectrophotometric colorimeter which is provided with a sheet polarizer S on the light incident side and a sheet polarizer P on the light receiving side, the reflection light of the pigment which is incident to the pearl pigment as a measurement sample at an angle of 45° to the direction normal to the surface of the sample and is received in the direction normal to the surface of the sample has absolute values a* and b* of 5 or less, and
wherein the average surface roughness of the coating is 10 nm or less,
wherein the flaky powder is mica, sericite, talc, kaolin, smectite clay mineral, synthetic mica, synthetic sericite, platelet-shaped titanium dioxide, platelet-shaped silica, platelet-shaped aluminium oxide, boron nitride, barium sulfate or platelet-shaped titania-silica composite oxide,
wherein the colored metal is gold or copper and wherein the colored metal oxide is iron oxide, lower titanium oxide, copper oxide, cobalt oxide, chromium oxide or nickel oxide.

2. The pearl pigment according to claim 1, wherein the colored metal is gold or the colored metal oxide is iron oxide.

3. The pearl pigment according to claim 1 or 2, wherein the coating layer has an optical thickness of 250 nm or less.

4. The pearl pigment according to any one of claims 1 to 3, wherein the coating layer has an optical thickness of 120 to 210 nm.

5. The pearl pigment according to any one of claims 1 to 3, wherein the coating layer has an optical thickness of 15 to less than 120 nm.

6. A process for producing a pearl pigment comprising: adding an aqueous solution of a colored metal oxide precursor to an aqueous dispersion of a flaky powder so that the amount of metal ion is 5 X 10⁻⁴ to 12 X 10⁻⁴ mol/min per 100 g of the flaky powder; adding an alkaline solution to the mixed solution so that the pH of the mixed solution is adjusted to 5 to 8; separating the solid from the mixed solution; and baking the separated solid at 500 to 1000°C.

7. The process for producing a pearl pigment according to claim 6, wherein when the aqueous solution of the colored metal oxide precursor is added to the aqueous dispersion of the flaky powder, the temperature of the mixed solution is in the range of 50 to 100°C.

8. The process for producing a pearl pigment according to any one of claims 6 or 7, wherein when the aqueous solution of the colored metal oxide precursor is added to the aqueous dispersion of the flaky powder, the pH of the mixed solution is in the range of 2 to 4.

9. The process for producing a pearl pigment according to any one of claims 6 to 8, wherein the colored metal oxide precursor is an iron oxide precursor.

10. A coating, printing ink, plastics, ceramics, or glaze for glasses comprising the pearl pigment according to any one of claims 1 to 5.

11. Make-up cosmetics comprising the pearl pigment according to any one of claims 1 to 5.

12. The make-up cosmetics according to claim 11, further comprising a lubricant having a viscosity of 1000 mPa·s or less at 25°C.

## Patentansprüche

1. Perlglanzpigment, umfassend ein flockiges Pulver, das an der Oberfläche mit einem farbigen Metall oder einem farbigen Metalloxid beschichtet ist,
wobei, wenn das Perlglanzpigment in einer durchschnittlichen Menge von 0,05 mg/cm² auf die Oberfläche von schwarzem Kunstleder aufgebracht ist und die Intensität des Lichts, die von dem Perlglanzpigment reflektiert wird, unter Verwendung von Lichtquelle C mit einem 2°-Betrachtungswinkel mit einem spektrophotometrischen Kolorimeter gemessen wird, der mit einer Polarisatorfolie S auf der Lichteinfallsseite und einer Polarisatorfolie P auf der Lichtempfangsseite versehen ist, das Reflexionslicht des Pigments, das in einem Winkel von 45° zu der Richtung senkrecht zur Oberfläche der Probe auf das Perlglanzpigment als Messprobe einfällt und in der Richtung senkrecht zur Oberfläche der Probe empfangen wird, absolute Werte a* und b* von 5 oder weniger aufweist, und
wobei die mittlere Oberflächenrauheit der Beschichtung 10 nm oder weniger ist,
wobei das flockige Pulver Glimmer, Sericit, Talkum, Kaolin, Smektit-Tonmineral, synthetischer Glimmer, synthetischer Sericit, plättchenförmiges Titandioxid, plättchenförmiges Siliciumoxid, plättchenförmiges Aluminiumoxid, Bornitrid, Bariumsulfat oder plättchenförmiges Titanoxid-Siliciumoxid-Kompositoxid (titania-silica composite oxide) ist,
wobei das farbige Metall Gold oder Kupfer ist und wobei das farbige Metalloxid Eisenoxid, niederes Titanoxid, Kupferoxid, Kobaltoxid, Chromoxid oder Nickeloxid ist.

2. Perlglanzpigment gemäß Anspruch 1, wobei das farbige Metall Gold ist oder das farbige Metalloxid Eisenoxid ist.

3. Perlglanzpigment gemäß Anspruch 1 oder 2, wobei die Beschichtungsschicht eine optische Dicke von 250 nm oder weniger aufweist.

4. Perlglanzpigment gemäß einem der Ansprüche 1 bis 3, wobei die Beschichtungsschicht eine optische Dicke von 120 bis 210 nm aufweist.

5. Perlglanzpigment gemäß einem der Ansprüche 1 bis 3, wobei die Beschichtungsschicht eine optische Dicke von 15 bis weniger als 120 nm aufweist.

6. Verfahren zur Herstellung eines Perlglanzpigments, umfassend:
das Hinzufügen einer wässrigen Lösung eines farbigen Metalloxidvorläufers zu einer wässrigen Dispersion eines flockigen Pulvers, so dass die Menge an Metallionen 5 X 10⁻⁴ bis 12 X 10⁻⁴ mol/min pro 100 g des flockigen Pulver ist;
das Hinzufügen einer alkalischen Lösung zu der gemischten Lösung, so dass der pH-Wert der gemischten Lösung auf 5 bis 8 eingestellt wird;
das Abtrennen des Feststoffs von der gemischten Lösung; und
das Backen des abgetrennten Feststoffs bei 500 bis 1000°C.

7. Verfahren zur Herstellung eines Perlglanzpigments gemäß Anspruch 6, bei dem, wenn die wässrige Lösung des farbigen Metalloxidvorläufers zu der wässrigen Dispersion des flockigen Pulvers hinzugefügt wird, die Temperatur der gemischten Lösung im Bereich von 50 bis 100°C liegt.

8. Verfahren zur Herstellung eines Perlglanzpigments gemäß einem der Ansprüche 6 oder 7, bei dem, wenn die wässrige Lösung des farbigen Metalloxidvorläufers zu der wässrigen Dispersion des flockigen Pulvers hinzugefügt wird, der pH-Wert der gemischten Lösung im Bereich von 2 bis 4 ist.

9. Verfahren zur Herstellung eines Perlglanzpigments gemäß einem der Ansprüche 6 bis 8, bei dem der farbige Metalloxidvorläufer ein Eisenoxidvorläufer ist.

10. Beschichtung, Druckfarbe, Kunststoff, Keramik oder Glasur für Gläser, umfassend das Perlglanzpigment gemäß einem der Ansprüche 1 bis 5.

11. Make-up-Kosmetikum, umfassend das Perlglanzpigment gemäß einem der Ansprüche 1 bis 5.

12. Make-up-Kosmetikum gemäß Anspruch 11, zusätzlich umfassend ein Gleitmittel mit einer Viskosität von 1000 mPa·s oder weniger bei 25°C.

## Revendications

1. Pigment nacré comprenant une poudre floconneuse revêtue sur la surface avec un métal coloré ou un oxyde métallique coloré,
dans lequel lorsque le pigment nacré est appliqué sur la surface de cuir synthétique noir en une quantité moyenne de 0,05 mg/cm² et que l'intensité de la lumière réfléchie par le pigment nacré est mesurée en utilisant un illuminant C à un angle de visualisation de 2° avec un colorimètre spectrophotométrique qui est muni d'un polariseur à feuille de type S sur le côté incidence de lumière et d'un polariseur à feuille de type P sur le côté réception de lumière, la lumière de réflexion du pigment qui est incidente sur le pigment nacré en tant qu'échantillon de mesure à un angle de 45° par rapport à la direction normale à la surface de l'échantillon et est reçue dans la direction normale à la surface de l'échantillon a des valeurs absolues a* et b* de 5 ou moins, et
dans lequel la rugosité de surface moyenne du revêtement est de 10 nm ou moins,
dans lequel la poudre floconneuse est du mica, de la séricite, du talc, du kaolin, un minéral argileux de smectite, du mica synthétique, de la séricite synthétique, du dioxyde de titane en forme de plaquette, de la silice en forme de plaquette, de l'oxyde d'aluminium en forme de plaquette, du nitrure de bore, du sulfate de baryum ou de l'oxyde composite de silice-oxyde de titane en forme de plaquette,
dans lequel le métal coloré est de l'or ou du cuivre et dans lequel l'oxyde métallique coloré est de l'oxyde de fer, de l'oxyde de titane inférieur, de l'oxyde de cuivre, de l'oxyde de cobalt, de l'oxyde de chrome ou de l'oxyde de nickel.

2. Pigment nacré selon la revendication 1, dans lequel le métal coloré est de l'or ou l'oxyde métallique coloré est de l'oxyde de fer.

3. Pigment nacré selon la revendication 1 ou 2, dans lequel la couche de revêtement a une épaisseur optique de 250 nm ou moins.

4. Pigment nacré selon l'une quelconque des revendications 1 à 3, dans lequel la couche de revêtement a une épaisseur optique de 120 à 210 nm.

5. Pigment nacré selon l'une quelconque des revendications 1 à 3, dans lequel la couche de revêtement a une épaisseur optique de 15 à moins de 120 nm.

6. Processus pour produire un pigment nacré comprenant : l'ajout d'une solution aqueuse d'un précurseur d'oxyde métallique coloré à une dispersion aqueuse d'une poudre floconneuse de sorte que la quantité d'ion métallique est de 5 X 10⁻⁴ à 12 X 10⁻⁴ mole/min par 100 g de la poudre floconneuse ; l'ajout d'une solution alcaline à la solution mélangée de sorte que le pH de la solution mélangée est ajusté entre 5 et 8; la séparation du solide de la solution mélangée; et la cuisson du solide séparé à 500 à 1000 °C.

7. Processus pour produire un pigment nacré selon la revendication 6, dans lequel lorsque la solution aqueuse du précurseur d'oxyde métallique coloré est ajoutée à la dispersion aqueuse de la poudre floconneuse, la température de la solution mélangée est dans la plage de 50 à 100 °C.

8. Processus pour produire un pigment nacré selon l'une quelconque des revendications 6 ou 7, dans lequel lorsque la solution aqueuse du précurseur d'oxyde métallique coloré est ajoutée à la dispersion aqueuse de la poudre floconneuse, le pH de la solution mélangée est dans la plage de 2 à 4.

9. Processus pour produire un pigment nacré selon l'une quelconque des revendications 6 à 8, dans lequel le précurseur d'oxyde métallique coloré est un précurseur d'oxyde de fer.

10. Revêtement, encre d'impression, plastique, céramique, ou verre pour des lunettes comprenant le pigment nacré selon l'une quelconque des revendications précédentes 1 à 5.

11. Cosmétique pour maquillage comprenant le pigment nacré selon l'une quelconque des revendications 1 à 5.

12. Cosmétique pour maquillage selon la revendication 11, comprenant en outre un lubrifiant ayant une viscosité de 1000 mPa·s ou moins à 25 °C.
